# EUROPÄISCHE PATENTSCHRIFT

(11) **EP 0 711 595 B1**
(45) Veröffentlichungstag und Bekanntmachung des Hinweises auf die Patenterteilung: **14.01.1998**
(21) Anmeldenummer: 95117117.2
(22) Anmeldetag: 31.10.1995
(51) Int. Cl.: B01J 8/06, B01J 8/02

(54) **Reaktor zur Durchführung heterogenkatalysierter Gasphasenreaktionen**
Reactor for carrying out heterogenous catalytic reactions in gas phase
Réacteur pour l'exécution de réactions à la catalyse hétérogène en phase gazeuse

(30) Priorität: 08.11.1994 DE 4439807
(43) Veröffentlichungstag der Anmeldung: 15.05.1996
(73) Patentinhaber: BASF AKTIENGESELLSCHAFT, 67056 Ludwigshafen (DE)
(72) Erfinder: Deimling, Axel, Dr., D-67434 Neustadt (DE); Behling, Uwe, D-67165 Waldsee (DE)

(56) Entgegenhaltungen:
- WO-A-90/06807
- DE-A- 1 426 731
- DE-A- 3 804 390
- US-A- 2 042 922
- US-A- 2 345 423
- US-A- 3 212 862

## Beschreibung

Zur technischen Durchführung von heterogenkatalysierten Gasphasenreaktionen (z.B. Formaldehyd-, Schwefel- oder Styrol-Synthese) werden im allgemeinen Festbettreaktoren (Axial- oder Radialbettreaktoren; Hordenreaktoren; Rohrbündelreaktoren) verwendet. Die Zu- bzw. Abfuhr der anfallenden oder erforderlichen Reaktionswärme macht im Verbund mit einem solchen Reaktor meist eine größere Anzahl von Wärmetauschern erforderlich. Die einzelnen Apparate sind mit Rohrleitungen untereinander verbunden. Eine Darstellung der Technologie ist z.B. Ullmanns Encyklopädie der technischen Chemie, Ausgabe 1973, Bd. 3, S. 465 ff. zu entnehmen.

Danach ist es bekannt, aus mehreren Baugruppen kombinierte Apparate zu verwenden, in denen - in der Regel räumlich getrennt - die umzusetzenden Gase aufgeheizt, in einem Katalysatorbett zur Umsetzung gebracht und schließlich wieder abgekühlt werden, wobei die mitgebrachte Wärme zur Aufheizung der Frischgase verwendet wird.

Nach einem nicht vorveröffentlichten Vorschlag wird zur Durchführung einer endothermen Umsetzung in einem aus Ober-, Mittel- und Unterteil bestehenden Reaktor ein Heizgas zunächst zum Beheizen eines Rohrbündelreaktors verwendet, der den Katalysator enthält; bevor die Heizgase den Reaktor verlassen, werden sie aber noch zum Vorheizen des umzusetzenden Frischgases verwendet, was in einem auf den Reaktor aufgesetzten zweiten Rohrbündel geschieht. Die Heizgaszufuhr erfolgt über ein beide Apparate verbindendes Zentralrohr.

Die Frischgase ihrerseits werden vorher in einem dritten Rohrbündel mit den abziehenden Reaktionsprodukten im Gegenstrom vorgewärmt. Dieses dritte Rohrbündel bildet das Unterteil des Reaktionsapparates; die vorgewärmten Frischgase werden dem Rauchgasvorerhitzer über eine außen am Apparat vorbeigeführte Frischgasleitung zugeführt.

Diese Anordnung hat Nachteile; insbesondere läßt sie sich nicht sehr kompakt bauen und bedarf einer aufwendigen zusätzlichen Isolierung des Außenrohres.

Es ist Aufgabe der Erfindung, einen Apparat anzugeben, der eine einfache, kompakte Anordnung aufweist, die gegenüber der getrennten Aufstellung und Verbindung von Einzelapparaten erheblich geringere Investitionskosten erfordert.

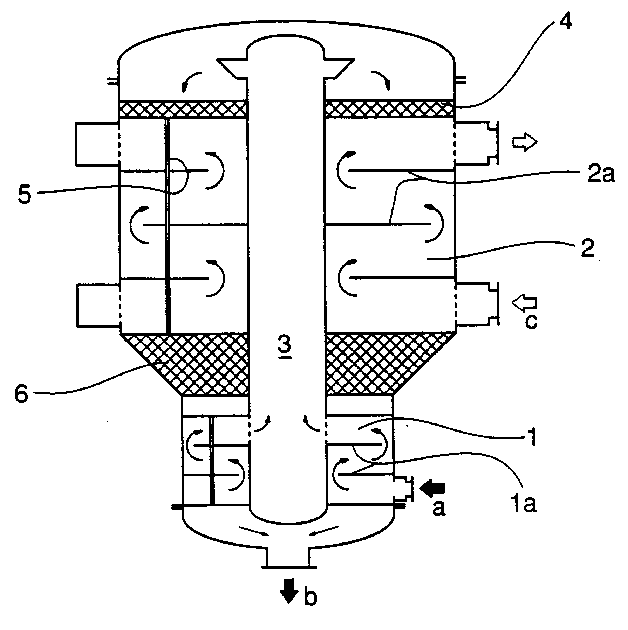

Der erfindungsgemäße Reaktor ist in der Figur dargestellt und kann zur Durchführung von exothermen oder - vorzugsweise - endothermen Umsetzungen dienen (von den nachstehend erwähnten Rohrbündeln ist jeweils nur ein Einzelrohr dargestellt). Das umzusetzende Frischgas (a) wird radial von außen nach innen in den Mantelraum eines Rohrbündelwärmetauschers 1 geleitet, der unterhalb des eigentlichen Reaktionsapparates 2/5 angeordnet ist. In diesem Wärmetauscher 1 kann eine Vorheizung des Frischgases dadurch geschehen, daß der Reaktionsaustrag (b), der von oben nach unten durch die Rohre strömt, abgekühlt wird. Nach Durchströmen des Wärmetauschers 1 gelangt das Frischgas in einen zylindrischen Innenraum 3, konzentrisch innerhalb des Reaktors selbst angeordnet, durch den es nach oben auf den Reaktor geleitet wird. Nach Umlenkung gelangt das Gas über eine bedarfsweise vorgeschaltete Katalysatorschicht 4 in die katalysatorgefüllten Rohres des Reaktionsapparats 2, in denen weitere Aufheizung und die Hauptumsetzung stattfindet.

Bei endothermen Umsetzungen wird die Reaktionswärme durch ein Heizgas (c) aufgebracht, welches im Gegenstrom zum Produktstrom durch den Mantelraum 2 um das Rohrbündel 5 des Reaktionsapparates geführt wird; wenn eine exotherme Umsetzung stattfinden soll, kann stattdessen ein Kühlmittel eingeleitet werden. Nach dem Verlassen des Rohrbündels 5 wird das Reaktionsgemisch zur schnellen Abkühlung noch durch eine nachgeschaltete Katalysatorschicht 6 geleitet, in der eine Nachreaktion stattfinden kann. In dem anschließenden Wärmetauscher 1 wird das Reaktionsgemisch unter Aufheizung des Frischgases abgekühlt. Bei exotherm ablaufenden Umsetzungen kann ein weiterer Wärmetauscher vorgesehen werden, der die Restwärme abführt.

Der erfindungsgemäße Reaktor kann demnach durch einen im wesentlichen fünfteiligen Aufbau beschrieben werden:

In einem gemeinsamen Mantel sind übereinander angeordnet
- ein seitlich angeströmter, mit Umlenkblechen 1a versehener Rohrbündelwärmetauscher 1 mit mehreren Einzelrohren;
- ein vorzugsweise konisch nach oben erweiterter, einen Boden aufweisender Reaktionsraum 6 zur Aufnahme einer ersten Katalysatorschüttung;
- ein seitlich angeströmter, mit Umlenkblechen 2a versehener zweiter Rohrbündelwärmetauscher 2 mit mehreren mit einem Katalysator befüllbaren Einzelrohren 5;
- ein einen Boden aufweisender, nach oben durch einen Gasdom abgeschlossener Reaktionsraum 4 zur Aufnahme einer zweiten Katalysatorschüttung;
- ein zentrales Rohr 3 zur Verbindung des Außenraums des ersten Rohrbündelwärmetauschers 1 mit dem Gasdom über dem Reaktionsraum 4;
sowie Mittel zum Ein- und Austritt eines Heiz- bzw. Kühlgases (c) in den bzw. aus dem zweiten Rohrbündelwärmetauscher und zum Eintritt (a) des Frischgases in den Mantelraum bzw. zum Austritt des Reaktionsgemisches aus den Rohren des ersten Rohrbündelwärmetauschers. Es versteht sich, daß die Erfindung nicht auf die Anordnung einschließlich der Katalysatorfüllung beschränkt ist.

### Beispiel

Zur Herstellung von Styrol wird dem vorstehend beschriebenen, katalysatorgefüllten Apparat ein Gemisch aus Wasserdampf und Ethylbenzol im Massenverhältnis von 1,25 : 1 zugeführt. Die Gesamtquerschnittsbelastung im Rohrteil beträgt 240 kmol/m²h (Dampf + Ethylbenzol). Es wird ein handelsüblicher Katalysator des Typs G 84 C (Hersteller: Südchemie) verwendet.

Die Rohre 5 des Hauptreaktors 2 haben einen Innendurchmesser von 72 mm und eine Länge von 5500 mm. Die Höhe der Schicht 6 beträgt 1200 mm, die der Schicht 4 ca. 500 mm. Der Manteldurchmesser des Reaktors im Bereich des Rohrbündels 5 beträgt 6800 mm.

Am Unterende der unbeheizten Katalysatorschicht 6 ist ein Ethylbenzolumsatz von 64 % erzielt. Die Styrolselektivität beträgt 95,5 mol-%. Der Druck am oberen Rohrboden beträgt ca. 1,8 bar_{abs}, nach der Schicht 6 ca. 0,5 bar_{abs}. Die Temperaturen des Katalysators und des Reaktionsgemisches im Apparat betragen am oberen Rohrboden (d.h. hinter der Schicht 4) ca. 420°C, am Ende des Rohrbündels 5 ca. 620°C und am Ende der Schicht 6 ca. 585°C. Das Heizgas hat am Eintritt eine Temperatur von ca. 720°C, am Austritt eine Temperatur von ca. 500°C.

## Patentansprüche

1. Reaktor zur Durchführung endothermer und/oder exothermer katalytisch geförderter Gasphasenreaktionen, der in einem gemeinsamen Mantel übereinander angeordnet die folgenden Bauelemente aufweist:
- einen seitlich angeströmten, mit Umlenkblechen (1a) versehenen ersten Rohrbündelwärmetauscher (1);
- einen vorzugsweise konisch nach oben erweiterten, einen Boden aufweisenden Reaktionsraum (6) zur Aufnahme einer ersten Katalysatorschüttung;
- einen seitlich angeströmten, mit Umlenkblechen (2a) versehenen zweiten Rohrbündelwärmetauscher (2) mit Rohren zur Aufnahme einer Katalysatorfüllung;
- einen einen Boden aufweisenden, nach oben durch einen Gasdom abgeschlossenen Reaktionsraum (4) zur Aufnahme einer zweiten Katalysatorschüttung;
ein zentrales Rohr (3) zur Verbindung des Außenraums des ersten Rohrbündelwärmetauschers (1) mit dem Gasdom über dem Reaktionsraum (4);
sowie Mittel zum Ein- und Austritt eines Heizgases (c) in bzw. aus dem zweiten Rohrbündelwärmetauscher und zum Eintritt (a) des Frischgases in den Mantelraum bzw. zum Austritt (b) des Reaktionsgemisches aus den Rohren des ersten Rohrbündelwärmetauschers.

## Claims

1. A reactor for carrying out endothermic and/or exothermic catalytically promoted gas-phase reactions, which has, arranged one on top of the other in a common jacket, the following components:
- a first tube-bundle heat exchanger (1) to which material flows laterally and which is provided with baffles (1a);
- a reaction space (6) which preferably widens conically upward, has a base and is intended for holding a first catalyst bed;
- a second tube-bundle heat exchanger (2) to which material flows laterally and which is provided with baffles (2a) and has tubes intended for holding a catalyst filling;
- a reaction space (4) which has a base, is closed at the top by a gas dome and is intended for holding a second catalyst bed;
- a central tube (3) for connecting the outer space of the first tube-bundle heat exchanger (1) to the gas dome above the reaction space (4);
and means for entry and exit of a heating gas (c) into and out of the second tube-bundle heat exchanger and means for entry (a) of the fresh gas into the jacket space or for exit (b) of the reaction mixture out of the tubes of the first tube-bundle heat exchanger.

## Revendications

1. Réacteur pour l'exécution de réactions endothermiques et/ou exothermiques catalysées en phase gazeuse, qui présente les éléments constitutifs suivants, superposés dans une chemise commune :
- un premier échangeur de chaleur à faisceau tubulaire (1), muni de chicanes (1a), à flux latéral;
- une chambre de réaction (6) de préférence conique élargie vers le haut, présentant un fond, pour recevoir un premier lit de catalyseur;
- un deuxième échangeur de chaleur à faisceau tubulaire (2), muni de chicanes (2a), à flux latéral, avec des tubes pour recevoir un remplissage de catalyseur;
- une chambre de réaction (4) présentant un fond, fermée sur le dessus par un dôme de gaz, pour recevoir un deuxième lit de catalyseur;
une conduite centrale (3) pour raccorder la chambre extérieure du premier échangeur de chaleur à faisceau tubulaire (1) au dôme de gaz au-dessus de la chambre de réaction (4);
de même que des moyens pour l'entrée et la sortie d'un gaz chauffant (c) dans le, respectivement hors du deuxième échangeur de chaleur à faisceau tubulaire et pour l'entrée (a) du gaz frais dans la chemise, respectivement pour la sortie (b) du mélange réactionnel des tubes du premier échangeur de chaleur à faisceau tubulaire.
